# EUROPEAN PATENT SPECIFICATION

(11) **EP 0 330 218 B1**
(45) Date of publication and mention of the grant of the patent: **25.01.1995**
(21) Application number: 89103247.6
(22) Date of filing: 24.02.1989
(51) Int. Cl.: A61K 31/13, A61K 31/135, A61K 31/38, C07C 211/24, C07C 215/24, C07C 323/23

(54) **Inhibitors of lysyl oxidase**
Lysyl-Oxidase-Inhibitoren
Inhibiteurs de la lysyle oxydase

(30) Priority: 25.02.1988 US 160364; 25.02.1988 US 160382
(43) Date of publication of application: 30.08.1989
(73) Proprietor: MERRELL DOW PHARMACEUTICALS INC., Cincinnati Ohio 45215-6300 (US)
(72) Inventor: Palfreyman, Michael G., Cincinnati Ohio 45249 (US); McDonald, Ian A., Loveland Ohio 45140 (US); Bey, Philippe, Cincinnati Ohio 45242 (US)
(74) Representative: Sgarbi, Renato

(56) References cited:
- US-A- 2 891 064
- US-A- 4 454 158
- US-A- 4 650 907
- JOURNAL OF ALLERGY AND CLINICAL IMMUNOLOGY, vol. 69, no. 3, March 1982, pages 297-305, The C.V. Mosby Co.; B. KANG et al.: "Successful treatment of far-advanced progressive systemic sclerosis by D-penicillamine"
- CHEMICAL ABSTRACTS, vol. 86, 1977, page 542, abstract no. 189096a, Columbus, Ohio, US; K. SCHULZE et al.: "Rearrangement of gamma-chloromethallyl thiocyanate to isomeric chlorovinyl isothiocyanate", & Z. CHEM. 1977, 17(2), 58-9
- The Merck Manual of Diagnosis and Therapy, 15th Edition, p. 1272-1290
- IIC, 17(5), 1986, 630-634

## Description

Collagen is the main protein of skin, tendon, bone, cartilage, and connective tissue. Collagen molecules are characterized by a triple-stranded helical structure made up of α-chains, and each collagen molecule is about 300 nm long and about 1.5 nm in diameter. Precursor pro-α-chains having certain residues called extension peptides not present in the final product, are the initial products of RNA mediated peptide synthesis within fibroblasts. These pro-α-chains first assemble into triple-stranded procollagen molecules intracellularly and the component lysine and proline residues are hydroxylated and subsequently glycosylated. During secretion, the extension peptides of the procollagen molecules are cleaved and collagen is formed. After secretion, collagen assembles into microfibrils and ultimately fibrils.

Strength of collagen is provided by crosslinking between various lysine residues both within a fibril and between fibrils. The first step of the crosslinking process is the deamination of lysine and hydroxylysine residues by extracellular lysyl oxidase to produce aldehyde groups. These highly reactive groups then form the crosslinks. The amount and type of crosslinking varies greatly according to the strength requirements of the various tissue types. If crosslinking is inhibited, the tissue becomes fragile and accordingly tears quite easily. Certain serious medical conditions are associated with the lack of collagen crosslinking such as Ehlers-Danlos Syndrome and Marfan's syndrome. While collagen crosslinking is essential, in certain instances it is desirable to prevent or reduce crosslinking such as in conditions and diseases characterized by defects in collagen metabolism such as occurs in various fibrotic conditions, for example, lung fibrosis, as well as in proliferative vitreo retinopathy, surgical scarring, systemic sclerosis, scleroderma, and keloids.

Certain inhibitors of collagen crosslinking are known such as penicillamine and beta-aminopropionitrile (BAPN). BAPN is known to prevent crosslinking specifically because of its ability to inhibit lysyl oxidase. Both penicillamine and BAPN have been studied extensively in animals and in humans for their effects on conditions associated with the abnormal deposition of collagen. The applicants have now discovered that certain halogenated allyl amines are inhibitors of lysyl oxidase and are useful in the treatment of diseases and conditions associated with abnormal collagen deposition.

### SUMMARY OF THE INVENTION

Use of a compound of formula 1 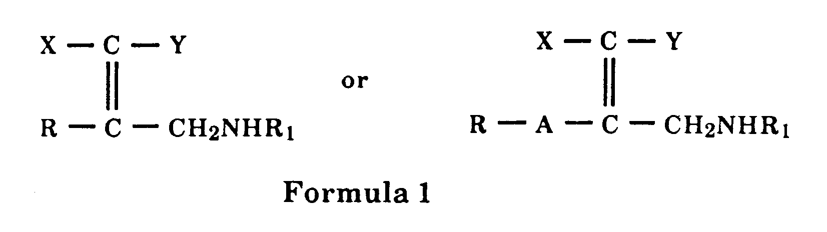
wherein
one of X and Y is a hydrogen and the other is a fluoro, chloro, or bromo group;
R₁ is a hydrogen, or a (C₁-C₄)alkyl group;
A is a divalent radical group selected from 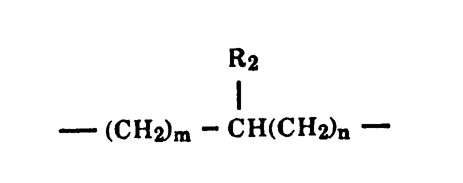
wherein
R₂ is hydrogen, methyl, or ethyl, and m and n, independently, are an integer from 0 to 16, provided that m + n cannot be greater than 17; and

-(CH₂)ₚ-D-(CH₂)_{q}-

wherein
D is oxygen or sulfur, p is an integer of from 0 to 16, and q is an integer of from 1 to 16, provided that p + q cannot be greater than 17;
R is a methyl group, a phenyl, or a phenyl substituted with one or two members of the group (C₁-C₅)alkyl, (C₁-C₅)alkoxy, hydroxy, chloro, bromo, fluoro, iodo, trifluoromethyl, nitro, or (C₁-C₅)alkylcarbonyl;
or
R is 2- or 3-thienyl;
or a pharmaceutically acceptable salt thereof, for preparing a medicament for treating diseases and conditions associated with the abnormal deposition of collagen such as they occur in fibrotic conditions, lung fibrosis, proliferative vitreo retinopathy, surgical scarring, systemic sclerosis, scleroderma, and keloids.

The present invention encompasses also a compound of the formula 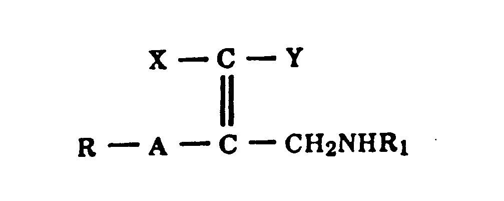
wherein
one of X and Y is a hydrogen and the other is a chloro or bromo group;
R₁ is a hydrogen or a (C₁-C₄)alkyl group;
A is a divalent radical group selected from 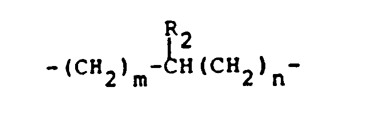
wherein
R₂ is hydrogen, methyl, or ethyl, and m and n, independently, are an integer from 0 to 16, provided that m + n cannot be greater than 17; and

-(CH₂)ₚ-D-(CH₂)_{q}-

wherein
D is oxygen or sulfur, p is an integer of from 0 to 16, and q is an integer of from 1 to 16, provided that p + q cannot be greater than 17; and
R is a methyl group;
or a pharmaceutically acceptable salt thereof.

### DETAILED DESCRIPTION OF THE INVENTION

Illustrative examples of divalent groups represented by A are -CH₂-, -(CH₂)₂-, -(CH₂)₃-, -(CH₂)₄-, -(CH₂)₅-, -CH₂S-(CH₂)₂-, and -CH₂O(CH₂)₂-. The term "(C₁-C₅)alkyl" means straight- and branched-chain alkyl groups. Illustrative examples of (C₁-C₅)alkyl groups are methyl, ethyl, n-propyl, isopropyl, n-butyl, isobutyl, tert-butyl, and n-pentyl. The term "(C₁-C₅)alkoxy" means straight-and branched-chain alkoxy groups. Illustrative examples of (C₁-C₅)alkoxy are methoxy, ethoxy, n-propoxy, isopropoxy, n-butoxy, isobutoxy, and n-pentoxy. The term "(C₁-C₅)alkylcarbonyl" means both straight- and branched-chain alkylcarbonyl groups. Examples of such groups are acetyl, propionyl, and n-butyryl.

It will be apparent to those skilled in the art that the compounds of Formula 1 contain one double bond, and therefore geometric isomerism is possible, i.e. at the allyl amine double bond. In naming the compounds of this invention the prefixes "(E)" and "(Z)" are used in the conventional manner to indicate stereochemistry at the double bond. If no stereochemical designation is given, both the substantially pure isomers or mixtures are intended. In those compounds wherein one of X and Y is a fluoro, chloro, or bromo group and the other is a hydrogen, applicants prefer those compounds wherein the halo group is oriented cis to the -R or -A-R group.

The compounds of this invention are useful both in the free base form and in the form of acid addition salts. The acid addition salts are simply a more convenient form for use and, in practice, use of the salt amounts to use of the free base. The expression "pharmaceutically acceptable acid addition salts" is intented to apply to any non-toxic organic or inorganic acid addition salts of the base compounds of formula 1. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the mono, di, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methanesulfonic acid and 2-hydroxyethanesulfonic acid. The salts can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic organic solvents and which in comparison to their free base forms, demonstrate higher melting points and an increased solubility.

Illustrative examples of the compounds of formula 1 are:
2-isobutyl-3-fluoro-, chloro-, or bromo-allylamine;
2-isopropyl-3-fluoro-, chloro-, or bromo-allylamine;
2-sec-butyl-3-fluoro-, chloro-, or bromo-allylamine;
2-butyl-3-fluoro-, chloro-, or bromo-allylamine;
2-hexyl-3-fluoro-, chloro-, or bromo-allylamine;
2-heptyl-3-fluoro-, chloro-, or bromo-allylamine;
2-ethoxymethyl-3-fluoro-, chloro-, or bromo-allylamine;
2-thioethoxymethyl-3-fluoro-, chloro-, or bromo-allylamine;
2-phenyl-3-fluoro-, chloro-, or bromo-allylamine;
2-(2-methoxyphenyl)-3-fluoro-, chloro-, or bromo-allylamine;
2-(3-methoxyphenyl)-3-fluoro-, chloro-, or bromo-allylamine;
2-(4-methoxyphenyl)-3-fluoro-, chloro-, or bromo-allylamine;
2-(2,3-dimethoxyphenyl)-3-fluoro-, chloro-, or bromo-allylamine;
2-(2,4-dimethoxyphenyl)-3-fluoro-, chloro-, or bromo-allylamine;
2-(2,5-dimethoxyphenyl)-3-fluoro-, chloro-, or bromo-allylamine;
2-(2,6-dimethoxyphenyl)-3-fluoro-, chloro-, or bromo-allylamine;
2-(3,4-dimethoxyphenyl)-3-fluoro-, chloro-, or bromo-allylamine;
2-(3,5-dimethoxyphenyl)-3-fluoro-, chloro-, or bromo-allylamine;
2-(2-hydroxyphenyl)-3-fluoro-, chloro-, or bromo-allylamine;
2-(3-hydroxyphenyl)-3-fluoro-, chloro-, or bromo-allylamine;
2-(4-hydroxyphenyl)-3-fluoro-, chloro-, or bromo-allylamine;
2-(2,3-dihydroxyphenyl)-3-fluoro-, chloro-, or bromo-allylamine;
2-(2,4-dihydroxyphenyl)-3-fluoro-, chloro-, or bromo-allylamine;
2-(2,5-dihydroxyphenyl)-3-fluoro-, chloro-, or bromo-allylamine;
2-(2,6-dihydroxyphenyl)-3-fluoro-, chloro-, or bromo-allylamine;
2-(3,4-dihydroxyphenyl)-3-fluoro-, chloro-, or bromo-allylamine;
2-(3,5-dihydroxyphenyl)-3-fluoro-, chloro-, or bromo-allylamine;
2-benzyl-3-fluoro-, chloro-, or bromo-allylamine;
2-phenethyl-3-fluoro-, chloro-, or bromo-allylamine;
2-(2-methoxybenzyl)-3-fluoro-, chloro-, or bromo-allylamine;
2-(3-methoxybenzyl)-3-fluoro-, chloro-, or bromo-allylamine;
2-(4-methoxybenzyl)-3-fluoro-, chloro-, or bromo-allylamine;
2-(2,3-dimethoxybenzyl)-3-fluoro-, chloro-, or bromo-allylamine;
2-(2,4-dimethoxybenzyl)-3-fluoro-, chloro-, or bromo-allylamine;
2-(2,5-dimethoxybenzyl)-3-fluoro-, chloro-, or bromo-allylamine;
2-(2,6-dimethoxybenzyl)-3-fluoro-, chloro-, or bromo-allylamine;
2-(3,4-dimethoxybenzyl)-3-fluoro-, chloro-, or bromo-allylamine;
2-(3,5-dimethoxybenzyl)-3-fluoro-, chloro-, or bromo-allylamine;
2-(2-hydroxybenzyl)-3-fluoro-, chloro-, or bromo-allylamine;
2-(3-hydroxybenzyl)-3-fluoro-, chloro-, or bromo-allylamine;
2-(4-hydroxybenzyl)-3-fluoro-, chloro-, or bromo-allylamine;
2-(2,3-dihydroxybenzyl)-3-fluoro-, chloro-, or bromo-allylamine;
2-(2,4-dihydroxybenzyl)-3-fluoro-, chloro-, or bromo-allylamine;
2-(2,5-dihydroxybenzyl)-3-fluoro-, chloro-, or bromo-allylamine;
2-(2,6-dihydroxybenzyl)-3-fluoro-, chloro-, or bromo-allylamine;
2-(3,4-dihydroxybenzyl)-3-fluoro-, chloro-, or bromo-allylamine;
2-(3,5-dihydroxybenzyl)-3-fluoro-, chloro-, or bromo-allylamine;
2-phenoxymethyl-3-fluoro-, chloro-, or bromo-allylamine;
2-(4-methoxyphenoxymethyl)-3-fluoro-, chloro-, or bromo-allylamine;
2-(2,4-dimethoxyphenoxymethyl)-3-fluoro-, chloro-, or bromo-allylamine;
2-(2,6-dimethoxyphenoxymethyl)-3-fluoro-, chloro-, or bromo-allylamine;
2-(3,4-methylenedioxyphenoxymethyl)-3-fluoro-, chloro-, or bromo-allylamine;
2-(3-hydroxyphenoxymethyl)-3-fluoro-, chloro-, or bromo-allylamine;
2-(4-hydroxyphenoxymethyl)-3-fluoro-, chloro-, or bromo-allylamine;
2-(2,3-dihydroxyphenoxymethyl)-3-fluoro-, chloro-, or bromo-allylamine;
2-(2,4-dihydroxyphenoxymethyl)-3-fluoro-, chloro-, or bromo-allylamine;
2-(2-methylphenoxymethyl)-3-fluoro-, chloro-, or bromo-allylamine;
2-(2-chlorophenoxymethyl)-3-fluoro-, chloro-, or bromo-allylamine;
2-(4-chlorophenoxymethyl)-3-fluoro-, chloro-, or bromo-allylamine;
2-(4-trifluoromethylphenoxymethyl)-3-fluoro-, chloro-, or bromo-allylamine;
2-thiophenoxymethyl-3-fluoro-, chloro-, or bromo-allylamine;
2-(4-methoxythiophenoxymethyl)-3-fluoro-, chloro-, or bromo-allylamine;
2-(2,4-dimethoxythiophenoxymethyl)-3-fluoro-, chloro-, or bromo-allylamine;
2-(2,4-dichlorothiophenoxymethyl)-3-fluoro-, chloro-, or bromo-allylamine;
2-(4-hydroxythiophenoxymethyl)-3-fluoro-, chloro-, or bromo-allylamine.

Presently preferred compounds of formula 1 are those in which one of X and Y is a chloro or bromo group.

Many of the compounds of this invention are known and the preparation of these compounds is described in, for example, United States Patent Number 4,454,158, granted June 12, 1984, British Patent Application Number 2,162,518, published February 5, 1986, and United States Patent Number 4,650,907, granted March 17, 1987. The preparation of those compounds not specifically described in these publications can readily be prepared using analogous procedures.

In particular, compounds of formula 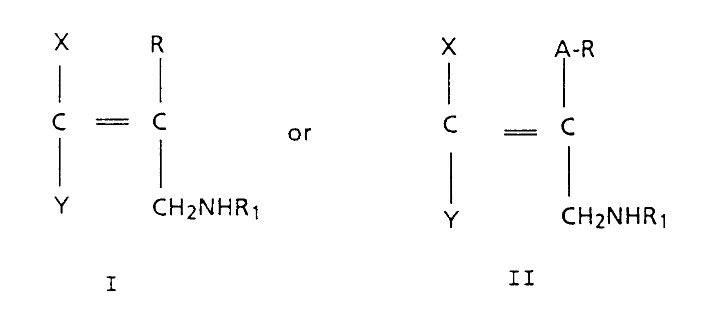
wherein
one of X or Y is hydrogen and the other is is chloro or bromo, R is methyl and R₁ and A are as defined above can be prepared by treating a compound of the formula 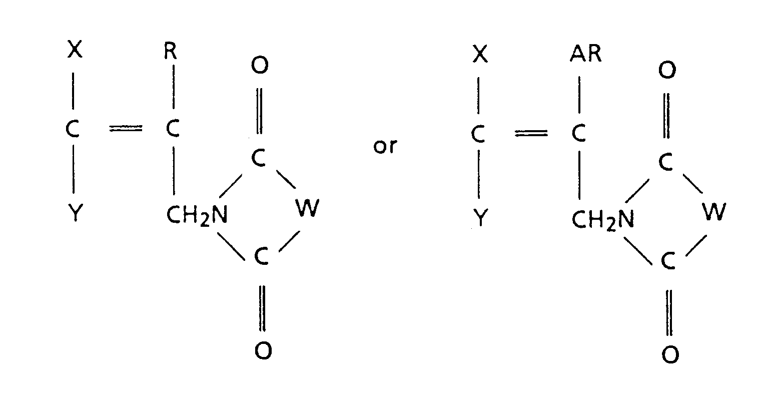
wherein X, Y, R, and A are as defined above and W is 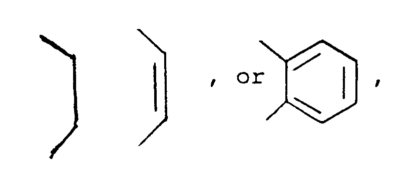
in a manner known per se to convert the succinimido, maleimido, or phthalimido group to the primary amino group.

A preferred method of preparation of those compounds of the above formula wherein W is 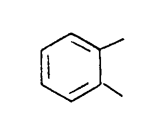
comprises a reaction with hydrazine or a hydrolytic cleavage using a strong mineral acid.

The ability of the compounds of this invention to be useful in the treatment of diseases and conditions associated with defects in collagen metabolism such as occurs in various fibrotic conditions, for example, lung fibrosis, as well as in proliferative vitreo retinopathy, surgical scarring, systemic sclerosis, scleroderma, and keloids can be demonstrated by the ability of the compounds to inhibit lysyl oxidase. The lysyl oxidase inhibition activity for representative members of the compounds of this invention is tabulated in Example 1.

The amount of the active ingredient to be administered can vary widely according to the particular dosage unit employed, the period of treatment, the age and sex of the patient treated and the nature and extent of the disorder treated. The total amount of the active ingredient to be administered will generally range from 5 mg to 500 mg per day. A unit dosage may contain from 25 to 500 mg of active ingredient, and can be taken one or more times per day. The active compound of formula 1 can be administered with a pharmaceutical carrier using conventional dosage unit forms either orally, parenterally, or topically. In the case of abnormal collagen deposition of the skin, topical administration to the diseased site is preferred, and in the case of abnormal collagen deposition to internal sites, local administration where possible and practical is preferred. Where local or topical application is not possible, systemic administration should be of short duration lasting, for example, for only a few days, and the patient should be closely monitored for adverse effects.

Coadministration of a compound of formula 1 with penicillamine, a compound known to be useful in the treatment of diseases and conditions characterized by abnormal collagen deposition but known to function by other than the inhibition of lysyl oxidase, is expected to be advantagous. The effective dosage of a compound of formula 1 when co-administered with penicillamine is expected to be less than the effective dosage when administered alone and will depend on the quantity and frequency of penicillamine co-administered. Therapy should be instituted at lower dosages of the formula 1 compound and of penicillamine than would be used in the absence of co-administration and the dosages thereafter altered to acheive the desired effect. The amount of compound of formula 1 as compared to the amount of penicillamine can vary from, for example, 1:1 to 1:500. It is understood that a compound of formula 1 can be administered substantially at the same time as, prior to, or after administration of penicillamine.

For oral administration the compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intented to assist the break-up and dissolution of the tablet following administration such as potato starch, alginic acid, corn starch, and guar gum, lubricants intented to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intented to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptable surfactant, suspending agent, or emulsifying agent.

The compounds of this invention may also be administered parenterally, that is, subcutaneously, intravenously, intramuscularly, or intraperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically acceptable adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions of this invention will typically contain from 0.5 to 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from 12 to 17. The quantity of surfactant in such formulations ranges from 5 to 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The compounds of this invention are preferably administered topically when used to treat a disease or condition characterized by abnormal collagen deposition of the skin. Any of the above described liquid formulations, including gels and ointments, may take the form of skin lotions and creams and may also contain emollients, perfumes, astringents, shaving lotions, colognes, cosmetic foundations, and similar preparations. In general a topical composition of this invention will contain from 0.01 g to 5 g of a compound of formula 1 per 100 ml of the composition.

### EXAMPLE 1

### LYSYL OXIDASE INHIBITION STUDIES

Lysyl oxidase preparation is obtained from bovine aorta by the procedures modified from M. A. Williams and H. M. Kagan, Anal. Biochem. 149, 430 - 437 (1985) and H. M. Kagan and K. A. Sullivan, Methods in Enzyml. 82, 637 - 650 (1982). The aorta is obtained fresh on the day of the enzyme preparation and is maintained at 4°C for the duration of its use in the experiments. The aorta is ground fine and is homogenized for 90 seconds in buffer (2.5 ml of a buffer consisting of 16 mM potassium phosphate and 1 mM phenylmethylsulfonyl fluoride/g of tissue) with 0.15 M NaCl added, then the mixture is centrifuged (20 minutes at 11,000 x g). The homogenization followed by centrifugation procedure is repeated with buffer plus 0.15 M NaCl, buffer alone, and buffer plus 1 M urea. After homogenization in 1 M urea, the mixture is stirred for 1 hour prior to centrifugation. The resulting pellet is homogenized in buffer plus 4 M urea, stirred for 18 hours, and centrifuged. The supernatant with lysyl oxidase activity is saved. The tissue is homogenized in buffer plus 4 M urea, stirred overnight, and centrifuged twice more. The supernatants with lysyl oxidase activity are saved.

The assay is adopted from that of P. C. Trackman, *et al*., Anal. Biochem. 113, 336 - 342 (1981). Each assay consists of two tubes, one that contains 0.2 mM β-aminopropionitrile, BAPN, from the start and one to which BAPN is added to quench the reaction. Lysyl oxidase preparation (0.150 ml), urea (0.300 ml, 4 M), buffer (0.930 ml, 200mM borate at pH = 8.2), homovanillic acid (0.020 ml of 50 mM solution), and horseradish peroxidase (0.010 ml of Sigma Type II at 5 mg/ml protein) are incubated for 2 minutes at 55°C in a test tube. Cadaverine (0.100 ml of 150 mM) and test compound, if any, is added and the incubation continued at 55°C for an additional 10 minutes. The test tubes containing this mixture are then cooled in a ice bath after adding BAPN to any tubes not containing it. The difference in fluorescence (excitation 315 nm and emission 425 nm) between corresponding tubes that received BAPN at 0 minutes and at 10 minutes is a measure of enzyme activity. A standard curve to determine the amount of cadaverine converted by lysyl oxidase is prepared as follows. Assay mixtures containing BAPN are made up as described and known amounts of hydrogen peroxide are added simultaneously with cadaverine and the fluorescence changes after 10 minutes reaction are determined.

Using this method the lysyl oxidase inhibiting activity expressed as IC₅₀ (inhibitory concentration), that is the concentration of test compound required to inhibit the enzyme activity by 50 per cent, was determined for various compounds of this invention. The results are shown in Table 1.

**TABLE 1**

| **LYSYL OXIDASE INHIBITION ACTIVITY OF HALOGENATED ALLYL AMINES** | |
|---|---|
| **TEST COMPOUND** | **IC₅₀ (M)** |
| (E)-2-(4-Fluorophenethyl)-3-fluoroallylamine | 1 x 10⁻¹⁰ |
| (E)-2-(3,4-Dimethoxyphenylpropyl)-3-fluoroallylamine | 1x10⁻¹⁰ |
| (E)-2-(Thiophenoxymethyl)-3-fluoroallylamine | 1x10⁻¹⁰ |
| (E)-2-Phenyl-3-fluoroallylamine | 1x10⁻⁹ |
| (Z)-2-(3,4-Dichlorophenoxymethyl)-3-fluoroallylamine | 1x10⁻⁹ |
| (Z)-2-(2-Thienyl)-3-fluoroallylamine | 1x10⁻⁹ |
| (E)-2-Isobutyl-3-fluoroallylamine | 1x10⁻⁸ |
| (E)-2-Undecyl-3-fluoroallylamine | 1x10⁻⁷ |
| (E)-2-(3,4-Dimethoxyphenyl)-3-fluoroallylamine | 1x10⁻⁷ |
| (E)-N-Ethyl-2-(3,4-dimethoxyphenyl)-3-fluoroallylamine | 1x10⁻⁶ |
| (E)-2-Phenethyl-3-chloroallylamine | 1x10⁻⁸ |
| (E)-2-Isobutyl-3-chloroallylamine | 1x10⁻⁷ |
| (E)-2-Phenyl-3-chloroallylamine | 1x10⁻⁷ |

### Preparation of 3-chloro-2-isobutylallylamine from the corresponding phthalimide

N-(3-chloro-2-isobutylallyl)-phthalimide (26.5 g) was suspended in 300 ml of ethanol and hydrazine monohydrate (5.26 g) was added. This reaction mixture was heated to reflux. After about 20 min, a solid appeared in the reaction medium. The mixture was refluxed for a total of 4 hrs. Then, it was cooled and HCl (6N, 260 ml) was added, and the resulting mixture was refluxed for 1 hr. Then, it was cooled to room temperature and the solvent was stripped off. The residue was allowed to stand overnight in the refrigerator. Then it was suspended in H₂O and filtered. The H₂O was evaporated off. The residue was suspended in toluene and the solvent evaporated off. The obtained residue was suspended in ethanol (dry) and the solvent was evaporated off to give 18.5 g of a white solid corresponding to the compound of the title.

### Conversion of 3-chloro-2-isobutylallylalcohol to N-(3-chloro-2-isobutylallyl)-phthalimide via 3-chloro-2-isobutylallylbromide

The starting material (3-chloroisobutylallylalcohol) (13.85 g) was dissolved in 150 ml of CH₂Cl₂ and cooled in an ice bath. PBr₃ (11.5 g) in 100 ml of CH₂Cl₂ was added dropwise. After the addition was completed, the reaction mixture was warmed to room temperature and stirred for 3.5 hours. Then, it was poured onto a saturated K₂CO₃ solution and the layers were separated. The organic layer was dried over MgSO₄ and concentrated to yield 15.0 g of a clear oil corresponding to 3-chloro-2-isobutylallylbromide.
This product was dissolved in 150 ml of DMF. Potassium phthalimide (13.14 g) was added and the reaction mixture was heated for 4 hours at 80°C then allowed to stand at room temperature overnight. Afterwards, the reaction mass was poured onto H₂O and extracted with ethyl ether. The ether layer was washed with 4% aqueous NaOH and subsequently with water (2 times). The ether layer was dried over MgSO₄ and concentrated to yield 14.0 g of a white solid corresponding to the compound of the title.

### Preparation of 3-chloro-2-isobutylallylalcohol

3-chloro-2-isobutyl acrylic acid ethyl ester (16.77 g) was dissolved in 150 ml of hexane and cooled on an ice bath. Then diisobutylaluminum hydride (DIBAL) (19 ml) was added by syringe. After the addition was complete the reaction mass was warmed to room temperature and stirred for 1 hour. A TLC assay indicated that the starting material was gone. The reaction mixture was then cooled in an ice bath and methanol (194 ml) was added. After about half of the addition was completed, the reaction mass which solidified was broken up and the addition of methanol was completed. HCl (270 ml, 6N) was then added, the solid dissolved and the layers were separated. The organic layer was dried over MgSO₄ and concentrated to yield 13.9 g of a clear oil, corresponding to the product of the title.

## Claims (Claims for the following Contracting State(s): DE, GB, FR, IT, NL, SE, CH, LI, BE, AT, LU)

1. Use of a compound of the formula 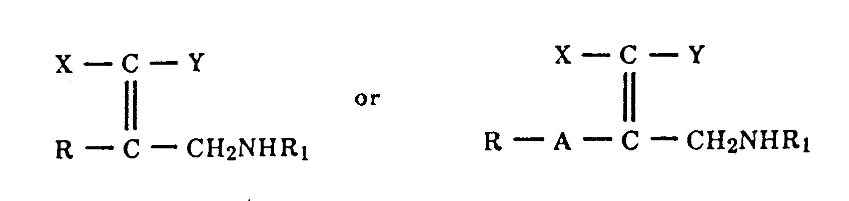 wherein
one of X and Y is a hydrogen and the other is a fluoro, chloro, or bromo;
R₁ is a hydrogen or a (C₁-C₄)alkyl group;
A is a divalent radical group selected from 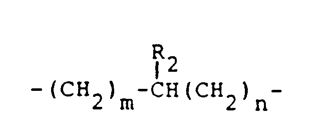 wherein
R₂ is hydrogen, methyl, or ethyl, and m and n, independently, are an integer from 0 to 16, provided that m + n cannot be greater than 17; and
-(CH₂)ₚ-D-(CH₂)_{q}-
wherein
D is oxygen or sulfur, p is an integer of from 0 to 16, and q is an integer of from 1 to 16, provided that p + q cannot be greater than 17;
R is a methyl group, a phenyl, or a phenyl substituted with one or two members of the group (C₁-C₅)alkyl, (C₁-C₅)alkoxy, hydroxy, chloro, bromo, fluoro, iodo, trifluoromethyl, nitro, or (C₁-C₅)alkylcarbonyl; or
R is 2- or 3-thienyl;
or a pharmaceutically acceptable salt thereof, for preparing a medicament for treating diseases and conditions associated with the abnormal deposition of collagen such as they occur in fibrotic conditions, lung fibrosis, proliferative vitreo retinopathy, surgical scarring, systemic sclerosis, scleroderma, and keloids.

2. A use according to claim 1 of a compound wherein one of X and Y is chloro or bromo and the other is a hydrogen.

3. A use according to any one of claims 1 or 2 of a compound wherein R is a phenyl or a phenyl mono- or di-substituted by (C₁-C₅)alkyl, (C₁-C₅)alkoxy, hydroxy, chloro, bromo, fluoro, iodo, trifluoromethyl, nitro, or (C₁-C₅)alkylcarbonyl.

4. A use according to any one of claims 1 or 2 of a compound wherein R is a methyl group and A, if present, is a divalent radical selected from 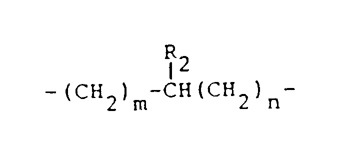 wherein
R₂ is hydrogen, methyl, or ethyl, and m and n, independently, are an integer from 0 to 16, provided that m + n cannot be greater than 17;
-(CH₂)ₚ-D-(CH₂)_{q}-
wherein
D is oxygen or sulfur, p is an integer of from 0 to 16, and q is an integer of from 1 to 16, provided that p + q cannot be greater than 17.

5. A use according to any one of claims 1 or 2 of a compound wherein R is a methyl group and A, if present, is a divalent radical selected from 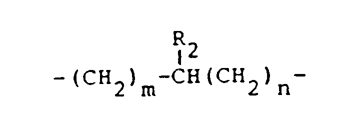 wherein
R₂ is hydrogen, methyl, or ethyl, m is an integer of from 0 to 16, and n is zero; and
-(CH₂)ₚ-D-(CH₂)_{q}-
wherein
D is oxygen or sulfur, p is an integer of from 0 to 16, and q is the integer 1.

6. A use according to claim 1 of a compound wherein one of X and Y is chloro or bromo and the other is hydrogen, and R is phenyl, methoxyphenyl, dimethoxyphenyl, hydroxyphenyl, dihydroxyphenyl, chlorophenyl or dichlorophenyl.

7. A use according to claim 1 of a compound wherein one of X and Y is chloro or bromo and the other is hydrogen and R is phenyl, methoxyphenyl, dimethoxyphenyl, hydroxyphenyl, dihydroxyphenyl, chldrophenyl or dichlorophenyl, and the group A is a methylene group or a group of the formulae -SCH₂- or -OCH₂-.

8. A product containing penicillamine and a compound of the formula 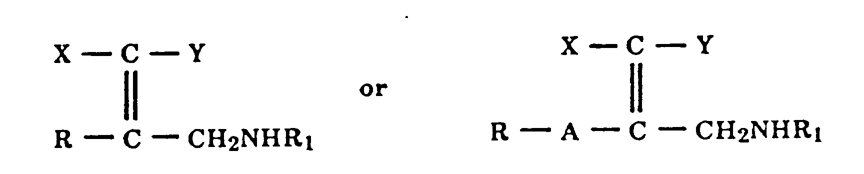 wherein
one of X and Y is a hydrogen and the other is a fluoro, chloro, or bromo;
R₁ is a hydrogen or a (C₁-C₄)alkyl group;
A is a divalent radical group selected from 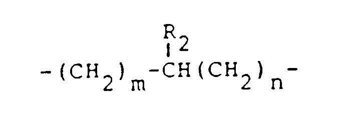 wherein
R₂ is hydrogen, methyl, or ethyl, and m and n, independently, are an integer from 0 to 16, provided that m + n cannot be greater than 17; and
-(CH₂)ₚ-D-(CH₂)_{q}-
wherein
D is oxygen or sulfur, p is an integer of from 0 to 16, and q is an integer of from 1 to 16, provided that p + q cannot be greater than 17;
R is a methyl group, a phenyl, or a phenyl substituted with one or two members of the group (C₁-C₅)alkyl, (C₁-C₅)alkoxy, hydroxy, chloro, bromo, fluoro, iodo, trifluoromethyl, nitro or (C₁-C₅)alkylcarbonyl; or
R is 2- or 3-thienyl;
or a pharmaceutically acceptable salt thereof, as a combined preparation for simultaneous, separate or sequential use in treating diseases and conditions associated with the abnormal deposition of collagen in a patient such as they occur in fibrotic conditions, lung fibrosis, proliferarive vitreo retinophaty, surgical scarring, systemic sclerosis, scleroderma and keloids.

9. A product according to claim 8 wherein the compound of the reported formula is one of any of claims 2 to 7.

10. A compound of the formula 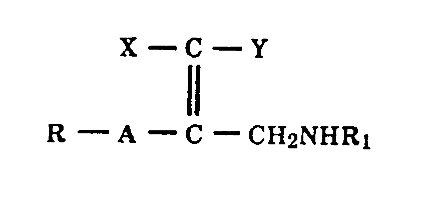 wherein
one of X and Y is a hydrogen and the other is a chloro or bromo group;
R₁ is a hydrogen or a (C₁-C₄)alkyl group;
A is a divalent radical group selected from 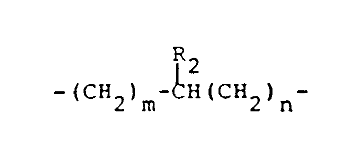 wherein
R₂ is hydrogen, methyl, or ethyl, and m and n, independently, are an integer from 0 to 16, provided that m + n cannot be greater than 17; and
-(CH₂)ₚ-D-(CH₂)_{q}-
wherein
D is oxygen or sulfur, p is an integer of from 0 to 16, and q is an integer of from 1 to 16, provided that p + q cannot be greater than 17; and
R is a methyl group;
or a pharmaceutically acceptable salt thereof.

11. A compound of claim 10 wherein A is a divalent radical selected from 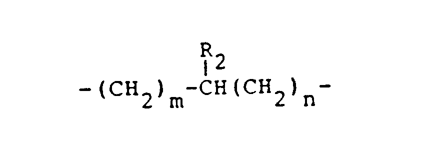 wherein
R₂ is hydrogen, methyl, or ethyl, and m and n, independently, are an integer from 0 to 16, provided that m + n cannot be greater than 17; and
-(CH₂)ₚ-D-(CH₂)_{q}-
wherein
D is oxygen or sulfur, p is an integer of from 0 to 16, and q is an integer of from 1 to 16, provided that p + q cannot be greater than 17.

12. A compound of claim 10 wherein A is a methylene group or a group of the formulae -SCH₂- or -OCH₂-.

13. The compound of claim 10 which is 2-chloromethylene-4-methyl-1-pentanamine.

14. The compound of claim 10 which is (E)-2-chloromethylene-4-methyl-1-pentanamine or a pharmaceutically acceptable salt thereof.

15. The compound of claim 14 which is (E)-2-chloromethylene-4-methyl-1-pentanamine, hydrochloride.

16. A pharmaceutical composition comprising a compound of any of claims 10 to 15 in admixture with a pharmaceutically acceptable carrier.

17. A compound of claims 10 to 15 for use as a medicine.

## Claims (Claims for the following Contracting State(s): ES, GR)

1. A process for preparing a compound of the formula 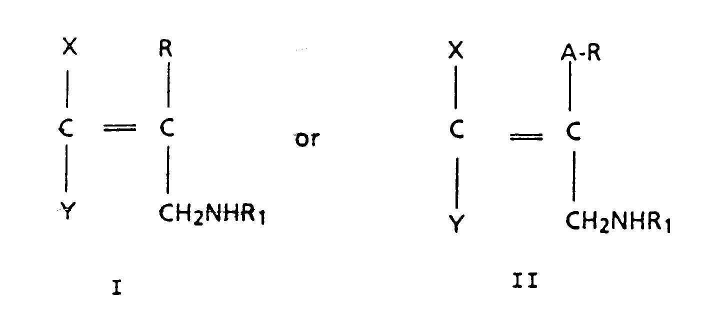 wherein
one of X and Y is a hydrogen and the other is a chloro or bromo group;
R₁ is a hydrogen or a (C₁-C₄)alkyl group;
A is a divalent radical group selected from 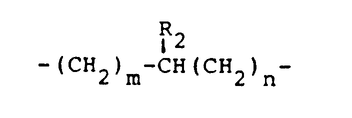 wherein
R₂ is hydrogen, methyl, or ethyl, and m and n, independently, are an integer from 0 to 16, provided that m + n cannot be greater than 17;
-(CH₂)ₚ-D-(CH₂)_{q}-
wherein
D is oxygen or sulfur, p is an integer of from 0 to 16, and q is an integer of from 1 to 16, provided that p + q cannot be greater than 17; and
R is a methyl group;
or a pharmaceutically acceptable salt thereof which comprises treating a compound of the formula 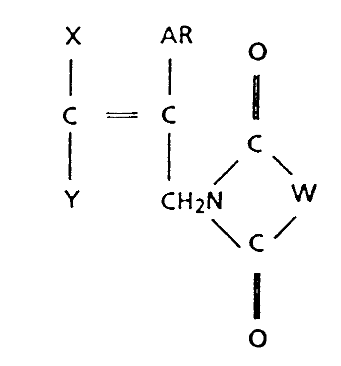 wherein X, Y, R, and A are as defined above and W is 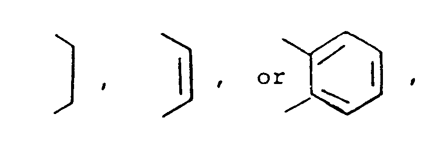 in a manner known per se to convert the succinimido, maleimido, or phthalimido group to the primary amino group.

2. A process according to claim 1 for preparing a compound wherein R₁ is hydrogen, methyl or ethyl and R, X, Y and A are as therein defined.

3. A process as claimed in claim 1 wherein W is 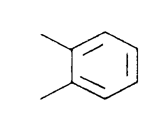 and the treatment comprises reaction with hydrazine or hydrolytic cleavage using a strong mineral acid.

4. A process according to claim 1 or any of the above claims for preparing a compound which is (E)-2-chloromethylene-4-methyl-1-pentanamine or an acid addition salt thereof.

5. A process according to claim 4 wherein the acid addition salt is the hydrochloride.

## Patentansprüche (Patentansprüche für folgende(n) Vertragsstaat(en): DE, GB, FR, IT, NL, SE, CH, LI, BE, AT, LU)

1. Verwendung einer Verbindung der Formel 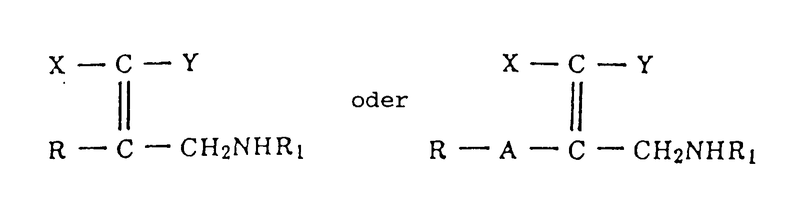 in der einer der Reste X und Y ein Wasserstoff- und der andere ein Fluor-, Chlor- oder Bromatom darstellt, R₁ ein Wasserstoffatom oder ein (C₁-C₄)-Alkylrest ist, A ein bivalenter Rest, ausgewählt aus 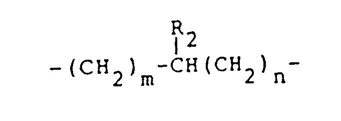 wobei R₂ ein Wasserstoffatom, eine Methyl- oder Ethylgruppe ist, und m und n unabhängig eine ganze Zahl von 0 bis 16 sind, mit der Maßgabe, daß m + n nicht größer als 17 sein kann, und
-(CH₂)ₚ-D-(CH₂)_{q}-
ist, wobei D ein Sauerstoff- oder Schwefelatom ist, p eine ganze Zahl von 0 bis 16 ist, und q eine ganze Zahl von 1 bis 16 ist, mit der Maßgabe, daß p + q nicht größer als 17 sein kann;
R eine Methyl-, Phenyl- oder mit ein oder zwei Mitgliedern aus der Gruppe (C₁-C₅)-Alkyl-, (C₁-C₅)-Alkoxyreste, Hydroxylgruppen, Chlor-, Brom-, Fluor- Jodatomen, Trifluormethyl-, Nitrogruppen oder (C₁-C₅)-Alkylcarbonylreste substituierte Phenylgruppe ist oder
R eine 2- oder 3-Thienylgruppe ist;
oder eines pharmazeutisch verträglichen Salzes davon, zur Herstellung eines Medikaments zur Behandlung von Erkrankungen und Leiden, die mit der abnormen Ablagerung von Collagen in Verbindung gebracht werden, wie sie bei fibrotischen Leiden, Lungenfibrose, wuchernder Glaskörperrethinopathie, operativer Narbenbildung, systemischer Sklerose, Sklerodermie und Keloiden auftreten.

2. Verwendung einer Verbindung nach Anspruch 1, in der einer der Reste X und Y ein Chlor- oder Bromatom und der andere ein Wasserstoffatom ist.

3. Verwendung einer Verbindung nach einem der Ansprüche 1 oder 2, in der R eine Phenyl- oder mit einem (C₁-C₅)-Alkyl-, (C₁-C₅)-Alkoxyrest, einer Hydroxylgruppe, einem Chlor-, Brom-, Fluor-, Jodatom, einer Trifluormethyl-, Nitrogruppe oder einem (C₁-C₅)-Alkylcarbonylrest mono- oder disubstituierte Phenylgruppe ist.

4. Verwendung einer Verbindung nach Anspruch 1 oder 2, in der R eine Methylgruppe ist und A, falls vorhanden, ein bivalenter Rest, ausgewählt aus 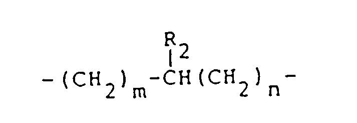 wobei R₂ ein Wasserstoffatom, eine Methyl- oder Ethylgruppe ist, und m und n unabhängig eine ganze Zahl von 0 bis 16 sind, mit der Maßgabe, daß m + n nicht größer als 17 sein kann, und
-(CH₂)ₚ-D-(CH₂)_{q}-
ist, wobei D ein Sauerstoff- oder Schwefelatom ist, p eine ganze Zahl von 0 bis 16 ist, und q eine ganze Zahl von 1 bis 16 ist, mit der Maßgabe, daß p + q nicht größer als 17 sein kann.

5. Verwendung einer Verbindung nach einem der Ansprüche 1 oder 2, in der R eine Methylgruppe ist, und A, falls vorhanden, ein bivalenter Rest, ausgewählt aus 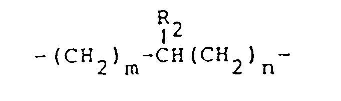 wobei R₂ ein Wasserstoffatom, eine Methyl- oder Ethylgruppe ist, m eine ganze Zahl von 0 bis 16 ist, und n null ist, und
-(CH₂)ₚ-D-(CH₂)_{q}-
ist, wobei D ein Sauerstoff- oder Schwefelatom ist, p eine ganze Zahl von 0 bis 16 ist, und q die Zahl 1 ist.

6. Verwendung einer Verbindung nach Anspruch 1, in der einer der Reste X und Y ein Chlor- oder Bromatom und der andere ein Wasserstoffatom ist, und R eine Phenyl-, Methoxyphenyl-, Dimethoxyphenyl-, Hydroxyphenyl-, Dihydroxyphenyl-, Chlorphenyl- oder Dichlorphenylgruppe ist.

7. Verwendung einer Verbindung nach Anspruch 1, in der einer der Reste X und Y ein Chlor- oder Bromatom und der andere ein Wasserstoffatom ist, und R eine Phenyl-, Methoxyphenyl-, Dimethoxyphenyl-, Hydroxyphenyl-, Dihydroxyphenyl-, Chlorphenyl- oder Dichlorphenylgruppe ist, und der Rest A eine Methylengruppe oder eine Gruppe der Formel -SCH₂- oder-OCH₂- ist.

8. Produkt, das Penicillamin und eine Verbindung der Formel 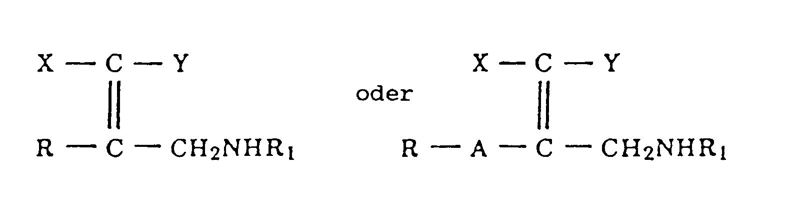 enthält, in der einer der Reste X und Y ein Wasserstoff- und der andere ein Fluor-, Chlor- oder Bromatom darstellt,
R₁ ein Wasserstoffatom oder ein (C₁-C₄)-Alkylrest ist,
A ein bivalenter Rest, ausgewählt aus 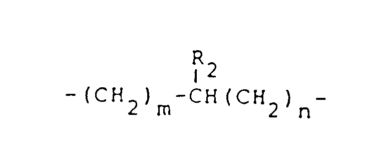 wobei R₂ ein Wasserstoffatom, eine Methyl- oder Ethylgruppe ist, und m und n unabhängig eine ganze Zahl von 0 bis 16 sind, mit der Maßgabe, daß m + n nicht größer als 17 sein kann, und
-(CH₂)ₚ-D-(CH₂)_{q}-
ist, wobei D ein Sauerstoff- oder Schwefelatom ist, p eine ganze Zahl von 0 bis 16 ist, und q eine ganze Zahl von 1 bis 16 ist, mit der Maßgabe, daß p + q nicht größer als 17 sein kann;
R eine Methyl-, Phenyl- oder mit ein oder zwei Mitgliedern aus der Gruppe (C₁-C₅)-Alkyl-, (C₁-C₅)-Alkoxyreste, Hydroxylgruppen, Chlor-, Brom-, Fluor- Jodatomen, Trifluormethyl-, Nitrogruppen oder (C₁-C₅)-Alkylcarbonylreste substituierte Phenylgruppe ist oder
R eine 2- oder 3-Thienylgruppe ist;
oder ein pharmazeutisch verträgliches Salz davon als kombiniertes Präparat zur gleichzeitigen, getrennten oder hintereinanderfolgenden Verwendung bei der Behandlung von Erkrankungen und Leiden, die mit der abnormen Ablagerung von Collagen bei einem Patienten in Verbindung gebracht werden, wie sie bei fibrotischen Leiden, Lungenfibrose, wuchernder Glaskörperrethinopathie, operativer Narbenbildung, systemischer Sklerose, Sklerodermie und Keloiden auftreten.

9. Produkt nach Anspruch 8, in dem die Verbindung mit der dargestellten Formel eine der Ansprüche 2 bis 7 ist.

10. Verbindung der Formel 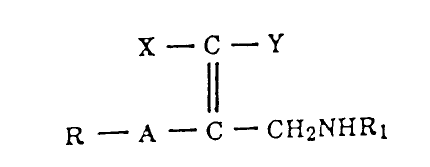 in der einer der Reste X und Y ein Wasserstoff- und der andere ein Chlor- oder Bromatom darstellt, R₁ ein Wasserstoffatom oder ein (C₁-C₄)-Alkylrest ist, A ein bivalenter Rest, ausgewählt aus 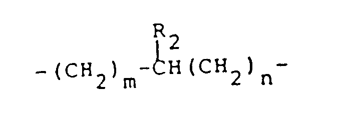 wobei R₂ ein Wasserstoffatom, eine Methyl- oder Ethylgruppe ist, und m und n unabhängig eine ganze Zahl von 0 bis 16 sind, mit der Maßgabe, daß m + n nicht größer als 17 sein kann, und
-(CH₂)ₚ-D-(CH₂)_{q}-
ist, wobei D ein Sauerstoff- oder Schwefelatom ist, p eine ganze Zahl von 0 bis 16 ist, und q eine ganze Zahl von 1 bis 16 ist, mit der Maßgabe, daß p + q nicht größer als 17 sein kann;
R eine Methylgruppe ist,
oder ein pharmazeutisch verträgliches Salz davon.

11. Verbindung nach Anspruch 10, in der A ein bivalenter Rest, ausgewählt aus 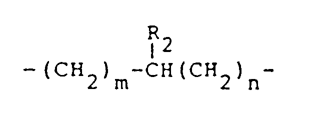 wobei R₂ ein Wasserstoffatom, eine Methyl- oder Ethylgruppe ist, und m und n unabhängig eine ganze Zahl von 0 bis 16 sind, mit der Maßgabe, daß m + n nicht größer als 17 sein kann, und
-(CH₂)ₚ-D-(CH₂)_{q}-
ist, wobei D ein Sauerstoff- oder Schwefelatom ist, p eine ganze Zahl von 0 bis 16 ist, und q eine ganze Zahl von 1 bis 16 ist, mit der Maßgabe, daß p + q nicht größer als 17 sein kann.

12. Verbindung nach Anspruch 10, in der A eine Methylengruppe oder eine Gruppe der Formel -SCH₂- oder -OCH₂-ist.

13. Verbindung nach Anspruch 10, nämlich 2-Chlormethylen-4-methyl-1-pentanamin.

14. Verbindung nach Anspruch 10, nämlich (E)-2-Chlormethylen-4-methyl-1-pentanamin oder ein pharmazeutisch verträgliches Salz davon.

15. Verbindung nach Anspruch 14, nämlich (E)-2-Chlormethylen-4-methyl-1-pentanaminhydrochlorid

16. Arzneimittel, umfassend eine Verbindung nach einem der Ansprüche 10 bis 15 in einem Gemisch mit einem pharmazeutisch verträglichen Träger.

17. Verbindung nach einem der Ansprüche 10 bis 15 zur Verwendung als Arznei.

## Patentansprüche (Patentansprüche für folgende(n) Vertragsstaat(en): ES, GR)

1. Verfahren zur Herstellung einer Verbindung der Formel 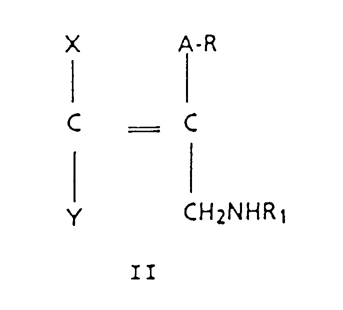 in der einer der Reste X und Y ein Wasserstoff- und der andere ein Chlor- oder Bromatom darstellt, R₁ ein Wasserstoffatom oder ein (C₁-C₄)-Alkylrest ist, A ein bivalenter Rest, ausgewählt aus 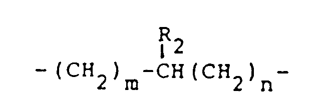 wobei R₂ ein Wasserstoffatom, eine Methyl- oder Ethylgruppe ist, und m und n unabhängig eine ganze Zahl von 0 bis 16 sind, mit der Maßgabe, daß m + n nicht größer als 17 sein kann, und
-(CH₂)ₚ-D-(CH₂)_{q}-
ist, wobei D ein Sauerstoff- oder Schwefelatom ist, p eine ganze Zahl von 0 bis 16 ist, und q eine ganze Zahl von 1 bis 16 ist, mit der Maßgabe, daß p + q nicht größer als 17 sein kann; und
R eine Methylgruppe ist;
oder eines pharmazeutisch verträglichen Salzes davon, umfassend die Behandlung einer Verbindung der Formel 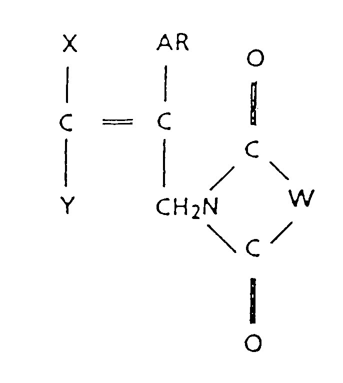 in der X, Y, R und A die vorstehend angegebenen Bedeutungen haben und W 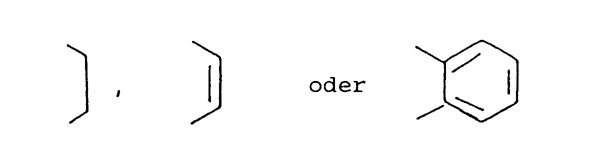 ist, auf an sich bekannte Weise, um die Succinimido-, Maleimido- oder Phthalimidogruppe in die primäre Aminogruppe umzuwandeln.

2. Verfahren nach Anspruch 1 zur Herstellung einer Verbindung, in der R₁ ein Wasserstoffatom, eine Methyl- oder Ethylgruppe ist, und R, X, Y und A die vorstehend angegebenen Bedeutungen haben.

3. Verfahren nach Anspruch 1, in der W 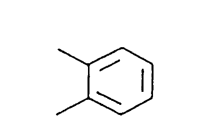 ist, und die Behandlung die Umsetzung mit Hydrazin oder eine hydrolytische Spaltung mit einer starken anorganischen Säure umfaßt.

4. Verfahren nach Anspruch 1 oder einem der vorstehenden Ansprüche zur Herstellung einer Verbindung, nämlich (E)-2-Chlormethylen-4-methyl-1-pentanamin oder eines Säureadditionssalzes davon.

5. Verfahren nach Anspruch 4, in dem das Säureadditionssalz das Hydrochlorid ist.

## Revendications (Revendications pour l'(les) Etat(s) contractant(s) suivant(s): DE, GB, FR, IT, NL, SE, CH, LI, BE, AT, LU)

1. Utilisation d'un composé de formule : 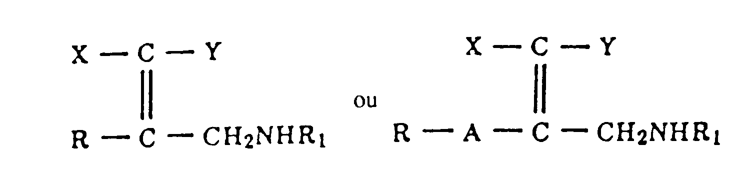 dans laquelle l'un des X et Y est un atome d'hydrogène et l'autre est un groupe fluoro, chloro ou bromo ;
R₁ est un atome d'hydrogène, ou un groupe (C₁-C₄)alkyle ;
A est un groupe radical divalent choisi parmi les suivants : 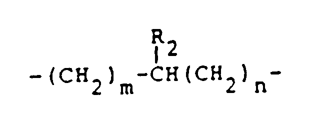 où R₂ est un atome d'hydrogène, un groupe méthyle ou éthyle et m et n, indépendamment, désignent un nombre entier de 0 à 16, à la condition que m + n ne soit pas supérieur à 17 ; et
-(CH₂)ₚ-D-(CH₂)_{q}-
où D est un atome d'oxygène ou de soufre, p est un nombre entier de 0 à 16, et q est un nombre entier de 1 à 16, à la condition que p + q ne soit pas supérieur à 17 ; R est un groupe méthyle, phényle ou phényle substitué par un ou deux éléments du groupe (C₁-C₅)alkyle, (C₁-C₅)alcoxy, hydroxy, chloro, bromo, fluoro, iodo, trifluorométhyle, nitro ou (C₁-C₅)alkylcarbonyle ; ou
R est un groupe 2- ou 3-thiényle ;
ou d'un sel pharmaceutiquement acceptable dudit composé, pour la préparation d'un médicament pour le traitement des maladies et des états associés à un dépôt anormal de collagène qui se produit par exemple dans les états fibrotiques, dans la fibrose pulmonaire, dans la vitréorétinopathie proliférative, dans la cicatrisation chirurgicale, dans la sclérose systémique, dans la sclérodermie et dans les chéloïdes.

2. Utilisation selon la revendication 1, d'un composé dans lequel l'un des X et Y est un groupe chloro ou bromo et l'autre est un atome d'hydrogène.

3. Utilisation selon l'une quelconque des revendications 1 et 2, d'un composé dans lequel R est un groupe phényle ou phényle mono- ou disubstitué par un groupe (C₁-C₅)alkyle, (C₁-C₅)alcoxy, hydroxy, chloro, bromo, fluoro, iodo, trifluorométhyle, nitro ou (C₁-C₅)alkylcarbonyle.

4. Utilisation selon l'une quelconque des revendications 1 et 2, d'un composé dans lequel R est un groupe méthyle et A, s'il est présent, est un radical divalent choisi parmi les suivants : 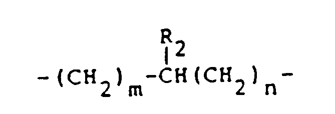 où R₂ est un atome d'hydrogène, un groupe méthyle ou éthyle et m et n, indépendamment, sont des nombres entiers de 0 à 16, à la condition que m + n ne soit pas supérieur à 17 ;
-(CH₂)ₚ-D-(CH₂)_{q}-
où D est un atome d'oxygène ou de soufre, p est un nombre entier de 0 à 16 et q est un nombre entier de 1 à 16, à la condition que p + q ne soit pas supérieur à 17.

5. Utilisation selon l'une quelconque des revendications 1 et 2, d'un composé dans lequel R est un groupe méthyle et A, s'il est présent, est un radical divalent choisi parmi les suivants : 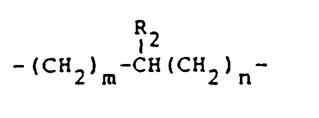 où R₂ est un atome d'hydrogène, un groupe méthyle ou éthyle, m est un nombre entier de 0 à 16, et n est égal à 0 ; et
-(CH₂)ₚ-D-(CH₂)_{q}-
où D est un atome d'oxygène ou de soufre, p est un nombre entier de 0 à 16 et q est un nombre entier égal à 1.

6. Utilisation selon la revendication 1, d'un composé dans lequel l'un des X et Y est un groupe chloro ou bromo et l'autre est un atome d'hydrogène, et R est un groupe phényle, méthoxyphényle, diméthoxyphényle, hydroxyphényle, dihydroxyphényle, chlorophényle ou dichlorophényle.

7. Utilisation selon la revendication 1, d'un composé dans lequel l'un des X et Y est un groupe chloro ou bromo et l'autre est un atome d'hydrogène et R est un groupe phényle, méthoxyphényle, diméthoxyphényle, hydroxyphényle, dihydroxyphényle, chlorophényle ou dichlorophényle et le groupe A est un groupe méthylène ou un groupe de formules -SCH₂- ou -OCH₂-.

8. Un produit contenant la pénicillamine et un composé de formule : 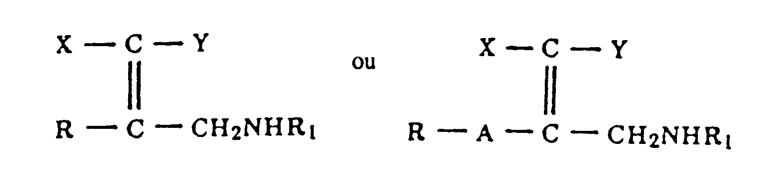 dans laquelle l'un des X et Y est un atome d'hydrogène et l'autre est un groupe fluoro, chloro ou bromo ;
R₁ est un atome d'hydrogène, ou un groupe (C₁-C₄)alkyle ;
A est un groupe radical divalent choisi parmi les suivants : 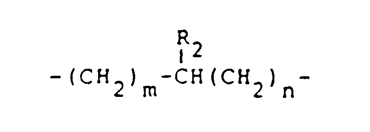 où R₂ est un atome d'hydrogène, un groupe méthyle ou éthyle et m et n, indépendamment, sont des nombres entiers de 0 à 16, à la condition que m + n ne soit pas supérieur à 17 ; et
-(CH₂)ₚ-D-(CH₂)_{q}-
où D est un atome d'oxygène ou de soufre, p est un nombre entier de 0 à 16, et q est un nombre entier de 1 à 16, à la condition que p + q ne soit pas supérieur à 17 ; R est un groupe méthyle, phényle ou phényle substitué par un ou deux éléments choisis parmi les groupes (C₁-C₅)alkyle, (C₁-C₅)alcoxy, hydroxy, chloro, bromo, fluoro, iodo, trifluorométhyle, nitro ou (C₁ -C₅)alkylcarbonyle ; ou
R est le groupe 2- ou 3-thiényle ;
ou d'un sel pharmaceutiquement acceptable dudit composé, sous la forme d'une préparation combinée pour l'utilisation simultanée, séparée ou séquentielle dans le traitement des maladies et des états associés à un dépôt anormal de collagène chez un patient qui se produit par exemple dans les états fibrotiques, la fibrose pulmonaire, la vitréorétinopathie proliférative, la cicatrisation chirurgicale, la sclérose systémique, la sclérodermie et les chéloïdes.

9. Un produit selon la revendication 8, selon laquelle le composé de formule rapportée est l'un quelconque des composés des revendications 2 à 7.

10. Un composé de formule suivante : 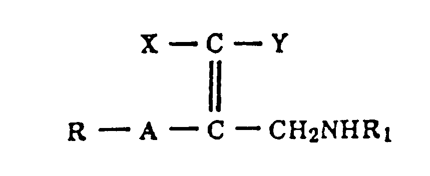 dans laquelle l'un des X et Y est un atome d'hydrogène et l'autre est un groupe chloro ou bromo ;
R₁ est un atome d'hydrogène ou un groupe (C₁-C₄)alkyle ;
A est un groupe radical divalent choisi parmi les suivants : 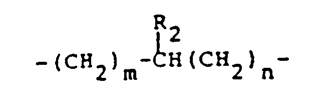 où R₂ est un atome d'hydrogène, un groupe méthyle ou éthyle et m et n, indépendamment, sont des nombres entiers de 0 à 16, à la condition que m + n ne soit pas supérieur à 17 ; et
-(CH₂)ₚ-D-(CH₂)_{q}-
où D est un atome d'oxygène ou de soufre, p est un nombre entier de 0 à 16, et q est un nombre entier de 1 à 16, à la condition que p + q ne soit pas supérieur à 17 ; et
R est un groupe méthyle ;
ou un sel pharmaceutiquement acceptable dudit composé.

11. Un composé selon la revendication 10, selon laquelle A est un radical divalent choisi parmi les suivants : 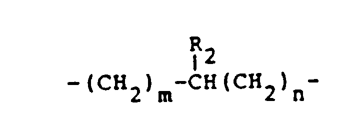 où R₂ est un atome d'hydrogène, un groupe méthyle ou éthyle et m et n, indépendamment, sont des nombres entiers de 0 à 10, à la condition que m + n ne soit pas supérieur à 17 ; et
-(CH₂)ₚ-D-(CH₂)_{q}-
où D est un atome d'oxygène ou de soufre, p est un nombre entier de 0 à 16, et q est un nombre entier de 1 à 16, à la condition que p + q ne soit pas supérieur à 17.

12. Un composé selon la revendication 10, selon laquelle A est un groupe méthylène ou un groupe de formules -SCH₂- ou -OCH₂-.

13. Le composé selon la revendication 10, qui est la 2-chlorométhylène-4-méthyl-1-pentanamine.

14. Le composé selon la revendication 10, qui est la (E)-2-chlorométhylène-4-méthyl-1-pentanamine ou un sel pharmaceutiquement acceptable dudit composé.

15. Le composé selon la revendication 14, qui est le chlorhydrate de (E)-2-chlorométhylène-4-méthyl-1-pentanamine.

16. Une composition pharmaceutique comprenant un composé selon l'une quelconque des revendications 10 à 15, en mélange avec un support pharmaceutiquement acceptable.

17. Un composé selon les revendications 10 à 15, à utiliser comme médicament.

## Revendications (Revendications pour l'(les) Etat(s) contractant(s) suivant(s): ES, GR)

1. Un procédé de préparation d'un composé de formule : 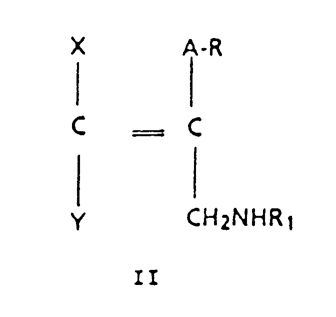 dans laquelle l'un des X et Y est un atome d'hydrogène et l'autre est un groupe chloro ou bromo ;
R₁ est un atome d'hydrogène ou un groupe (C₁-C₄)alkyle ;
A est un groupe radical divalent choisi parmi les suivants : 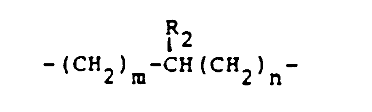 où R₂ est un atome d'hydrogène, un groupe méthyle ou éthyle et m et n, indépendamment, sont des nombres entiers de 0 à 16, à la condition que m + n ne soit pas supérieur à 17 ; et
-(CH₂)ₚ-D-(CH₂)_{q}-
où D est un atome d'oxygène ou de soufre, p est un nombre entier de 0 à 16, et q est un nombre entier de 1 à 16, à la condition que p + q ne soit pas supérieur à 17 ;
et
R est un groupe méthyle ;
ou d'un sel pharmaceutiquement acceptable dudit composé, qui comprend le traitement d'un composé de formule : 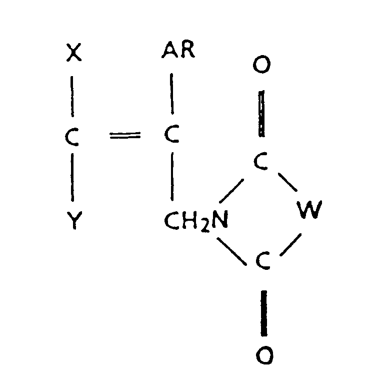 dans laquelle X, Y, R et A sont comme définis précédemment et W est 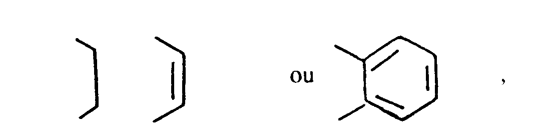 de manière connue per se pour convertir le groupe succinimido, maléimido ou phtalimido en un groupe amino primaire.

2. Un procédé selon la revendication 1, pour la préparation d'un composé dans lequel R₁ est un atome d'hydrogène, un groupe méthyle ou éthyle et R, X, Y et A sont comme définis ci-dessus.

3. Un procédé selon la revendication 1, selon laquelle W est 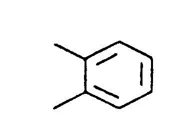 et le traitement comprend la réaction avec l'hydrazine ou le clivage hydrolytique utilisant un acide minéral fort.

4. Un procédé selon la revendication 1 ou l'une quelconque des revendications précédentes pour la préparation d'un composé qui est la (E)-2-chlorométhylène-4-méthyl-1-pentanamine ou un sel d'addition d'acide de ce composé.

5. Un procédé selon la revendication 4, selon laquelle le sel d'addition d'acide est le chlorhydrate.
